# EUROPEAN PATENT SPECIFICATION

(11) **EP 3 398 443 B1**
(45) Date of publication and mention of the grant of the patent: **15.01.2020**
(21) Application number: 17169048.0
(22) Date of filing: 02.05.2017
(51) Int. Cl.: A23G 1/00, A23G 1/18, G01N 25/04, G01N 33/02

(54) **APPARATUS FOR DETERMINING CRYSTALLISATION SOLIDIFICATION CURVES OF CHOCOLATE MASS AND OTHER FAT MASSES**
VORRICHTUNG ZUR BESTIMMUNG DER KRISTALLISATIONSERSTARRUNGSKURVEN VON SCHOKOLADENMASSE UND ANDEREN FETTMASSEN
APPAREIL PERMETTANT DE DÉTERMINER DES COURBES DE SOLIDIFICATION DE LA CRISTALLISATION D'UN MÉLANGE DE CHOCOLAT ET D'AUTRES MÉLANGES ADIPEUX

(43) Date of publication of application: 07.11.2018
(73) Proprietor: Aasted ApS, 3520 Farum (DK)
(72) Inventor: HOLMUD, Dennis, 2860 Søborg (DK)
(74) Representative: Heiden, Finn

(56) References cited:
- EP-A1- 1 591 786
- DE-A1- 4 209 073
- DE-C1- 3 714 951
- DE-U1-202011 100 655

## Description

The present invention is defined by the claims and concerns a unit according to at least claim 1, the unit comprising an apparatus for determining crystallization solidification curves of chocolate mass and other fat masses and a supply conduit part with flanges, the apparatus being arranged onto an opening in the supply conduit part with the flanges adapted to be installed in a chocolate mass conduit for tempered chocolate mass from a tempering apparatus, comprising an outer housing part arranged at the outer periphery of the supply conduit part and an inner housing part extending in through the opening in radial direction towards the center of the conduit part, in which conduit part the end of the inner housing part is arranged, a channel extending in radial direction from the outer housing and into the inner housing, a piston arranged in the channel, a temperature sensor arranged in the piston, a pressure source arranged at the outer housing part adapted for controlling the movements of the piston, when a sample of chocolate mass is to be drawn into a measurement chamber in the channel or when the sample is to be exposed after temperature measurement, a tempering source adapted for cooling of the inner housing part under temperature measurement and solidification of the sample in the chamber, and a stop arranged in the outer housing for determining the outer position of the piston in the channel thereby providing the measurement chamber in the channel between the piston in the outer position and the end of the inner housing part. According to the invention, the tempering source, which is adapted for both cooling and heating, comprises at least one Peltier element.

Such apparatus is often called a Tempermeter within the field of chocolate tempering. Generally, chocolate mass and other fat mass tempered encompass all types of suspensions of non-fat particles such as sugar, milk powders and cocoa solids mixed up with a liquid fat constituent, so that the suspensions are capable of crystallizing. It could be chocolate types used in any kind of production of chocolate articles or it could be masses such as creme mass used inside articles as filling, upon or as "sandwiching" layers in articles in both production of bakery articles as well as of chocolate articles. When it comes to the most widely used chocolate mass types, the fat constituent comprises genuine cocoa butter typically in a content of up to approximately 35%. However, the fat phase may also comprise substitutes as well. A small content of up to 2-3 % of genuine cocoa butter may still be left in the recipe. Substitutes may be in the form of other types of fat-containing oils such as palm-kernel oil. Chocolate types having the cocoa butter been replaced by other fats are often named commercially as compound chocolate, especially when the cocoa butter has been replaced completely by palm-kernel oil. Mass made of up to 100% cocoa butter may however also be continuously tempered. It is used later as constituent in the production of different chocolate mass recipes.

To the satisfaction of the manufacturer, it is decisive, that whether the fat phase constitutes of genuine cocoa butter or substitutes, the fat phase must be capable of crystallizing into stable crystal types, such as the βV-crystals developing in genuine cocoa butter. However, it is important to avoid instable crystals in the solidified mass. Only then, chocolate articles with good taste, crisp break and shiny appearance are created. The solidified chocolate articles will also achieve the longest possible shelf life and the best resistance against bloom, as instable crystals are diminished. If there is a content of in-stable crystals left in the mass, they will give rise to shorter shelf-life as the articles will bloom more quickly as when in-stable crystals are not present.

For the manufacturers it is of outmost importance that the tempered mass delivered to the production of articles is having a content of the stable crystal-type only, such as βV-crystals in chocolate mass. Only then, the manufacturer can rely on, that the quality of his chocolate products is consistent.

For this purpose, an apparatus of the introductory art is used, which during the solidification of the sample in the measurement chamber measures the temperature of the sample mass continuously or intermittently with time. A so-called "Temper curve" is then produced, from which a person skilled in the art of chocolate tempering can conclude the degree of tempering of the mass. The form of the curvature is an expression for the heat released by the particular types of crystals created in the mass sample during solidification with time. On this basis is determined if un-stable crystals are present. The so-called "Tempering Index" may also be calculated by a computer as a value indicating the degree of crystallisation of the desired crystals.

The operator can then adjust the tempering apparatus until the tempered mass is satisfactory after repetitive controls of samples. Adjustment may also be done automatically by a computer targeting at a certain tempering index.

A device of the type described in the beginning is known from the DE 37 14 951 C1 known. A measuring chamber is provided, which is enclosed by a cooled wall. The measuring chamber is connected at a removal point for liquid pre-crystallized chocolate, for example to a conduit section from which the liquid chocolate for the sample is removed. The measuring chamber partially projects into the flow cross-section of the conduit. The measuring chamber itself has a cylindrical cross-section and is thus matched to a cylindrical piston which is displaceable in the measuring chamber. The piston carries a temperature sensor which protrudes from the front wall of the piston and projects into the measuring chamber in the retracted position of the piston and traces and receives the temperature of the solidifying sample. The measuring chamber is thus formed by a piston / cylinder unit, which is arranged with its open-end face immersed in the chocolate material to be measured. A drive for the stroke of the piston is provided, which is dimensioned approximately to the length of the measuring chamber. Further, means for removing the solidified sample from the plunger and the temperature sensor is provided. This device consists of a mechanical comminution device, a milling and cutting device, the axis of which is arranged in alignment with the axis of the measuring chamber. The milling or cutting device has a shaft driven by a motor and a milling head which projects into the flow cross-section of the measuring point from the outside and partially blocks the flow cross-section. The projecting shaft must be sealed.

EP1591786 discloses another apparatus of the introductory art, by which the inner housing is extending all the way through the conduit and into a second outer housing at the opposite side of the conduit. In the second housing is arranged a melting chamber. The solidified sample is then pushed by the action of the piston from the measuring chamber, through the conduit to the opposite side of the conduit and into the melting chamber from which it is exposed into the mass in the conduit after being melted. At the retraction of the piston mass is sucked into the inner housing and is filling out the measuring chamber ready for the next solidification and temperature measuring. The apparatus has many parts and requires a modification of the chocolate mass conduits on both sides thereof. It takes time and requires skills to arrange the apparatus parts correctly through the conduit and montage the housing parts correctly at both opposite sides of the conduit. The arrangement at both sides also requires that space is available all around the conduit.

DE4209073 discloses a further apparatus by which the measuring chamber is arranged completely outside the conduit of tempered mass. At the outer end of the chamber is arranged a membrane valve. When the valve is opened, the mass in the conduit flow into the measurement chamber where it is being solidified by cooling. After being solidified during temperature measurement the solidified sample is melted directly in the chamber and being exposed therefrom to the outside of the conduit. The system is open to the outside which is not recommended in today's production environments. It requires manual control of the valve and the steps of cooling and heating as well as removal of samples outside the conduit.

DE 20 2011 100 655 U1 discloses a further apparatus for determining crystallisation curves of chocolate mass by which the measuring chamber is arranged completely outside the periphery of the chocolate conduit. The measuring chamber is connected with the inner of the chocolate conduit via a small opening through which the mass is pumped. The apparatus comprises a a housing with a channel through which a piston is slidably movable by means of pneumatic pressure and in which a temperature sensor is centrally and coaxially mounted. The tip of the sensor slides forth and back through the small opening. The housing comprises an end face which closes the channel apart from the central opening. The measuring chamber is formed by the maximum distance reached by the piston when drawing mass from the conduit. The apparatus further comprises means for cooling and heating in direct contact with the wall of the measurement chamber, which could be Peltier Elements. The piston draws a sample of mass into the measuring chamber. The sample is cooled and solidified when being measured. Thereafter the sample is heated and exposed by the pistion through the small opening into the flow of mass in the chocolate conduit. As the mass is being drawn outside the chocolate conduit through the small opening and furthermore has to be returned through the small opening after measurement it is likely to assume that a measuring cycle is time-consuming. For the chocolate manufacturer longer time periods of uncertainty can be costly as it takes more time before undesirable tempering stings can be adjusted so that an erroneous production may be corrected. The apparatus is then not especially effective.

The inventive apparatus is characterized in that the end of the inner housing part comprises a stern part closing off the channel apart from a central opening fitted for the movement of the temperature sensor in the opening when moving the piston forth and back in radial direction, and that a plurality of openings are arranged around the inner housing part for flow of chocolate mass between the conduit part and the measurement chamber in the channel, and that the tempering source is also adapted for heating of the inner housing part, so that a solidified sample in the measurement chamber can be melted before being exposed by the piston through the plurality of openings, and that the tempering source, which is adapted for both cooling and heating is comprising at least one Peltier Element.

When the piston is retracted to the outer position at the stop, liquid, tempered chocolate in the conduit effectively flows through the plurality of channels in the inner housing part and into the measurement chamber where the measurement begins as the inner housing is cooled by the influence of the tempering source in the outer housing. Effectively a solid layer of chocolate is soon created at the inside of the inner housing thereby providing a sort of shell which protects the still liquid sample in the inner of the chamber against the heat from the chocolate flow in the conduit.

During increasing solidification, the temperature of the sample is measured by the sensor in the piston. When the chocolate sample in the measurement chamber has solidified, the tempering source is activated to begin heating of the inner housing until the chocolate sample is soft or even liquid. The piston is then activated to travel in the channel to the inner position at the stern part at the same time squeezing the sample chocolate out through the plurality of openings in the inner housing so that the sample mixes nicely up into the flow of tempered chocolate mass in the conduit.

The inventive apparatus provides the available measurement chamber in the inner of the conduit part, so that the sample can be disposed fast and effectively out into the flow of chocolate in the conduit without the use of any further mechanical apparatus means or installations and without the necessity of moving the sample to another position before disposing it. The inventive solution is simple and performs fast and effectively.

It is especially surprising that the measurement chamber can be maintained fully in the flow of chocolate in the conduit as the sample is being cooled and temperature measured. Then the inventive solution provides the unique advantage that the sample can be heated in the same position and disposed directly out into flow of chocolate in the conduit, only by the return travel of the piston.

Thereby is provided an apparatus having an unforeseen effective combined measurement and melting chamber for the chocolate samples.

When the stop is arranged at a position in the outer housing, so that when the piston is at the stop in the outer position, the inner stern surface of the piston is at least at the inner surface of the conduit part or in the conduit part, thereby determining the full length of the measurement chamber to be within the conduit part. The full length of the measurement chamber is then arranged in the flow of the tempered chocolate in the conduit part. The flow of chocolate mass in the conduit then ensures that the chocolate flushing through the plurality of openings in the inner end of the housing will flow through the complete length of the measurement chamber. Then there will be no "dead" spots in the measurement chamber not being filled with fresh chocolate mass when the cooling of the sample begins. When the cooling begins all parts of the sample will then be exposed the same environmental influence from the temperature and flow of the tempered chocolate in the conduit. The inner housing with the plurality of openings is like a "Swiss cheese" when the cooling begins, however soon is a solidifying chocolate shell created at the inner housing surface protecting the inner still liquid sample against the temperature of the chocolate flow in the conduit during continued solidification of the sample.

When the temperature measurement is concluded and the sample being fully solidified, the heating starts and the complete length of the measurement chamber in the conduit ensures that the melting sample is easily disposed out into the flow of mass when the piston is moving to the inner position at the stern of the inner housing part.

When the full length of the measurement chamber is between % and ½ of the inner diameter of the conduit, there is an advantageous balance between the flushing effect through the plurality of openings in the inner housing part and the resistance the inner housing provides in the main flow of chocolate in the conduit. Arranging the length of the measurement chamber between % and ½ of the inner diameter of the conduit provides an effective flushing effect of the chocolate through the plurality of openings. On the other hand, the resistance exerted by the inner housing in the main flow of chocolate is still so moderate, that it is acceptable.

Especially advantageous is that the tempering source adapted for cooling and heating of the inner housing part is at least one Peltier-element. Electrical power to the Peltier-elements are advantageously controlled by a device such as a computer, so that pole reversing is performed when controlling from cooling to heating and vice versa. This makes the inventive apparatus especially fast when performing a full sample test of measurement and succeeding disposal of the sample.

When the at least one Peltier-element is arranged in direct contact with the inner housing part, the heat transfer is fast and effective. Advantageously may two or more Peltier-elements be arranged at opposite sides of the inner housing part.

The apparatus is especially fast and easy to fit-in and montage when it is arranged in a conduit part with flanges adapted to be installed in the chocolate mass conduit from the tempering apparatus.

When a flow diverging plate is arranged in the conduit part up-stream of the apparatus and is hinged at one end to an axel extending transversely through the conduit part and the opposite wall parts thereof, at least one end of the axel being adapted to be turned so as to position the flow diverging plate in a desired angular position, then the flow of tempered chocolate mass in the conduit is increased and directed towards the inner housing part. The increased flow is flushing even more effectively through the plurality of openings. This is especially an advantage when the chocolate mass has a high viscosity such as milk chocolate, white chocolate, nougat or filling mass for pralines or like mass with a high content of ingredients in more course particle form.

The invention is explained further below under reference to preferred embodiments as well as the drawing, in which
fig. 1 is a perspective view of the inventive apparatus for determining solidification curves of chocolate mass and other fat masses arranged on the conduit part with flanges adapted to be installed in a supply conduit for tempered chocolate mass from a tempering apparatus,
fig. 2 is a front view of the inventive apparatus of figure 1 assembled to the supply conduit of a tempering apparatus,
fig. 3 is the inventive apparatus of figure 1, seen in vertical cross section without chocolate in the measurement chamber,and without the flanges of the conduit part,
fig. 4 is a schematic view of the inventive apparatus of figures 1-3 connected with control devices, and installed in between the chocolate conduit of the tempering apparatus and the main chocolate conduit leading a continuous flow of chocolate to the manufacturing of articles,
fig. 5 is the same as in figure 3 with a chocolate sample in the measurement chamber,
fig. 6 is the same as in figure 3 and 5 having the chocolate sample being disposed from the measurement chamber, and
fig. 7 is a tempering curve for chocolate mass provided by the set-up in figure 4.

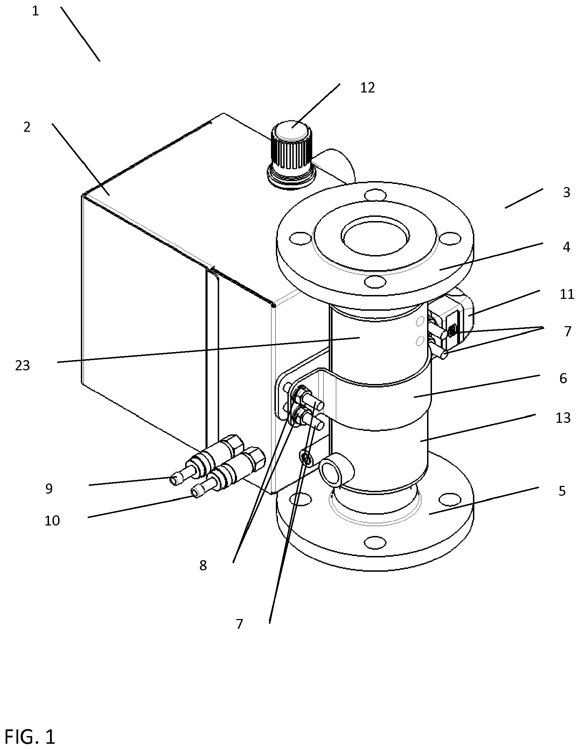

The apparatus 1 disclosed in figure 1 has a cover 2 and is fitted to the conduit part 3 having flanges 4, 5. A clamp 6 is arranged around the conduit part 3 and via bolts 7 and nuts 8 the clamp 6 and the conduit part 3 is kept to the apparatus 1. The apparatus has connections for pressurized air 9, 10, a multi-connection 11 for electrical power, data transmission of temperature measurements, control of Peltier-elements, etc. In this embodiment is also arranged a knob for the regulation of the pressurized air. There could be several more connections for measurements of movable parts in the apparatus and temperature of the housing itself. However, these are not part of the basic inventive idea and are as such not described further. The conduit part 3 is surrounded by a mantle 13 for circulation of water thereby ensuring the correct temperature conditions for the tempered chocolate passing through the conduit part 3.

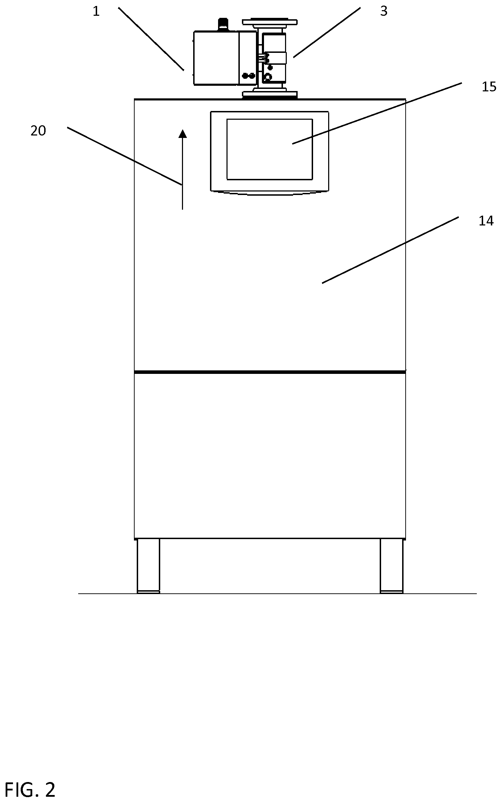

In figure 2 is disclosed the combined unit of the inventive measurement apparatus 1 and the conduit part 3 of figure 1 fitted onto a typical apparatus 14 for continuous tempering of chocolate mass in a high capacity production, which could be from 200 kg/hour to 10.000 kg/hour of tempered chocolate mass. A touch screen 15 is build-in at the front of the tempering apparatus for achieving the apparatus control. At the screen, it is also possible to control the inventive apparatus 1. However, the control of the apparatus 1 may also be performed by a computer such as a laptop or similar device.

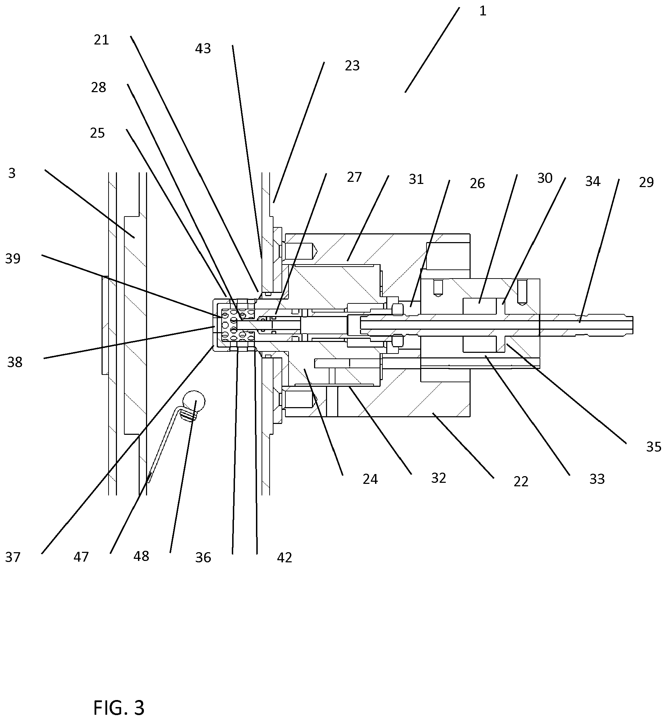

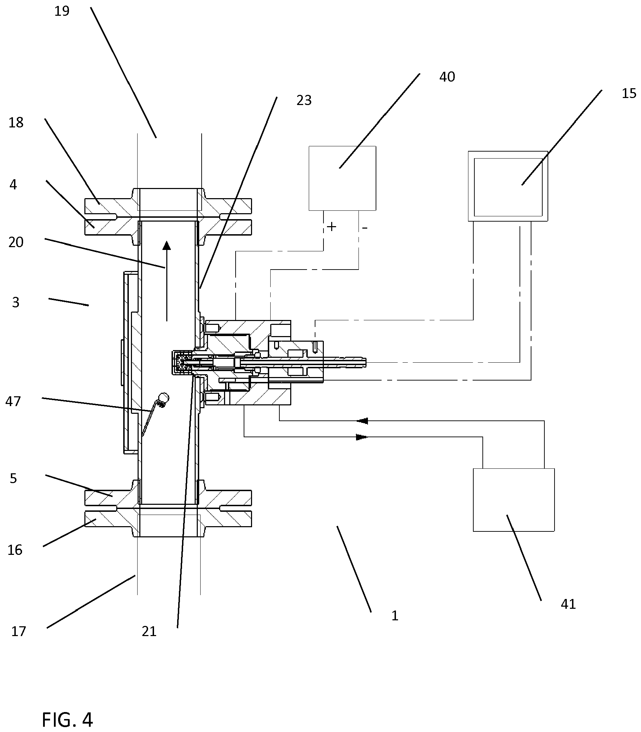

In figure 4 is schematically disclosed that the lower flange 5 of the conduit part 3 is fitted onto the upper flange 16 of the chocolate conduit 17 of the tempering apparatus 14. The flanges 5 and 16 are fitted together, usually by non-disclosed nuts and bolts through holes in the flanges. Schematically is also disclosed, that the upper flange 4 is fitted to a lower flange 18 of the preceding main chocolate conduit 19 leading a continuous flow of tempered chocolate mass to the manufacturing of articles. In both figure 3 and 4, the arrow 20 depicts the flow direction of the chocolate mass.

In figures 3-6 the apparatus 1 is depicted without the cover 2 for the sake of clarity. Only a smaller length of the conduit part 3 is disclosed as the flanges 4, 5 are not part of the inventive idea. The inventive apparatus 1 is arranged into an opening 21 in the conduit part 3, and that could be anywhere in a conduit supplying tempered chocolate in the production. The only modification necessary is to drill a hole in the conduit part 3 and clamp the inventive apparatus on to the conduit part 3.

The apparatus comprises an outer housing part 22 arranged at the outer periphery 23 of the supply conduit part 3 and an inner housing part 24 extending in through the opening 21 in radial direction in towards the center of the conduit part 3, in which conduit the end 25 of the inner housing part 24 is arranged. A channel 26 is extending in radial direction from the outer housing 22 and into the inner housing 24. A piston 27 is arranged in the channel 26. A temperature sensor 28 is arranged centrally in the piston 27. Wiring from the sensor 28 is not disclosed for clarity, however, which typically leaves the piston through a central bore 29.

A pressure source comprising pressurized air in a chamber 30 arranged at the outer housing part 22 adapted for controlling the movements of the piston 27. A tempering source in the form of an upper Peltier-element 31 and a lower Peltier-element 32 adapted for cooling as well as heating of the inner housing parts 24.

A stop 33 is arranged in the outer housing 22 for determining the outer position of the piston 27 in the channel 26. In the embodiment disclosed in the drawings the stop 33 is arranged between a radially extending ring part 34 at the piston 27 and an end surface 35 in the pressurized chamber 30. Thereby is provided a measurement chamber 36 between the piston 27 in the outer position and the end 25 of the inner housing part 24.

The end 25 of the inner housing part 24 comprises a stern part 37 closing off the channel 26 apart from a central opening 38 fitted for the movement of the temperature sensor 28 in the opening 38 when moving the piston 27 forth and back in radial direction.

A plurality of circular openings 39 are arranged evenly distributed around the inner housing part 24 for flow of chocolate mass between the measurement chamber 36 in the channel 26 and the conduit part 3.

An electrical power source 40 is connected to the Peltier-Elements 31, 32 and other parts in the apparatus that require electrical power. This is schematically disclosed in figure 4, as the skilled person know how to do such connections. The touch-screen or lap-top 15 is at least connected with the temperature sensor 28. All electrical connections are advantageously made via the multi-connection 11.

An external source of pressurized air 41 is in figure 4 schematically disclosed connected with the chamber 30 in the outer housing part 22. The source of pressurized air 41 may also be controlled by the touch-screen or lap-top 15.

Before a measurement process begins the piston 27 is retracted to the outer position at the stop 33. Liquid, tempered chocolate in the conduit 3 effectively flow through the plurality of openings 39 in the inner housing part 24 and into the measurement chamber 36.

In the disclosed embodiment of the drawings the stop 33 consists of a radially extending ring part 34 at the piston 27 and an end surface 35 in the pressurized chamber 30. The stop 33 is advantageously arranged so that the inner stern surface 42 of the piston 27 is at least at the inner surface 43 of the conduit part 3 or in the conduit part 3. The full length of the measurement chamber 36 is thereby within the conduit part 3 and arranged in the flow of the tempered chocolate in the conduit. The flow of chocolate mass in the conduit then ensures that the chocolate flushing through the plurality of openings in the inner end 25 of the housing 24 will flow through the complete length of the measurement chamber. Then there is no "dead" spots in the measurement chamber 36 not being filled with fresh chocolate mass when the cooling of the sample begins. Arranging the length of the measurement chamber between ¼ and ½ of the inner diameter of the conduit provides an effective flushing effect of the chocolate through the plurality of openings. On the other hand, the resistance exerted by the inner housing in the main flow of chocolate is still so moderate, that it is acceptable.

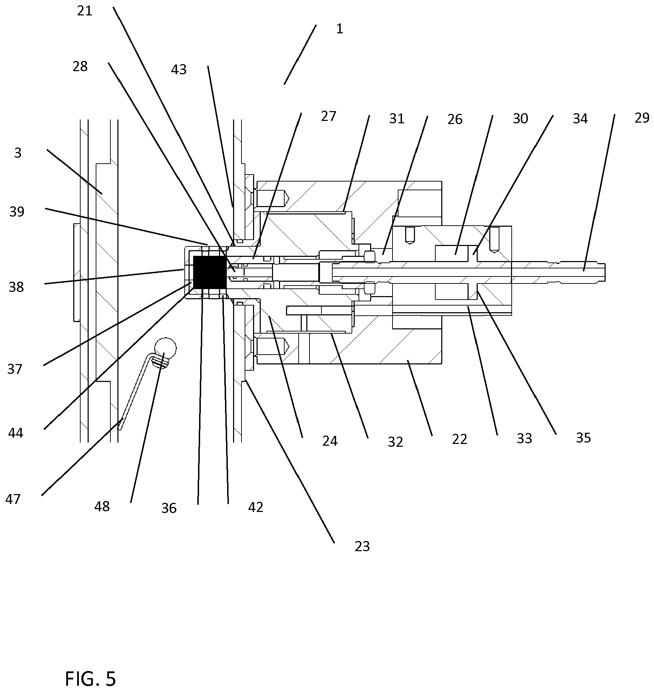

When the cooling begins all parts of the sample will then be exposed the same environmental influence from the temperature and flow of the tempered chocolate in the conduit. The inner housing 24 with the plurality of openings 39 is like a "Swiss cheese" when the cooling begins, however soon is a solidifying chocolate shell created at the inner housing surface protecting the inner still liquid sample against the temperature of the chocolate flow in the conduit during continued solidification of the sample 44 disclosed in figure 5.

The measurement begins as the inner housing part 24 is cooled by the influence of the two Peltier-elements 31, 32. Electrical power to the Peltier-elements 31, 32 are controlled by the touch-screen or computer 15, so that the electrical power source 40 provides the correct polarity and voltage over the Peltier-elements for cooling of the inner housing part 24. The heat transfer is fast and effective. Effectively a solid layer of chocolate is soon created at the inside 45 of the measurement chamber 36 in the inner housing 24 thereby providing a sort of shell which protects the still liquid sample in the inner of the chamber 36 against the warm from the chocolate flow in the conduit part 3.

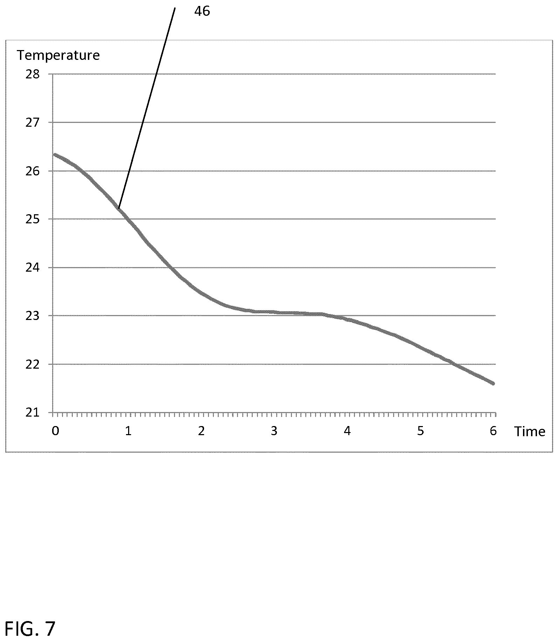

During increasing solidification, the temperature of the sample 44 is measured by the sensor 28 in the piston 27 and registered with regular time-intervals by the touch-screen or computer 15. When the chocolate sample 44 in the measurement chamber 36 has completely solidified, the complete "temperature with time" data is available for the particular tempered chocolate mass in the conduit. The data can be computed and presented, for example as a "Temper Curve" 46 as the one disclosed in figure 7. After evaluation adjustments of the tempering process of the particular mass in the conduit may be made if required. Either by automatically by the touch-screen or computer 15 or manually. The curve 46 represents a well-tempered chocolate mass which will be recognized by the skilled chocolate maker.

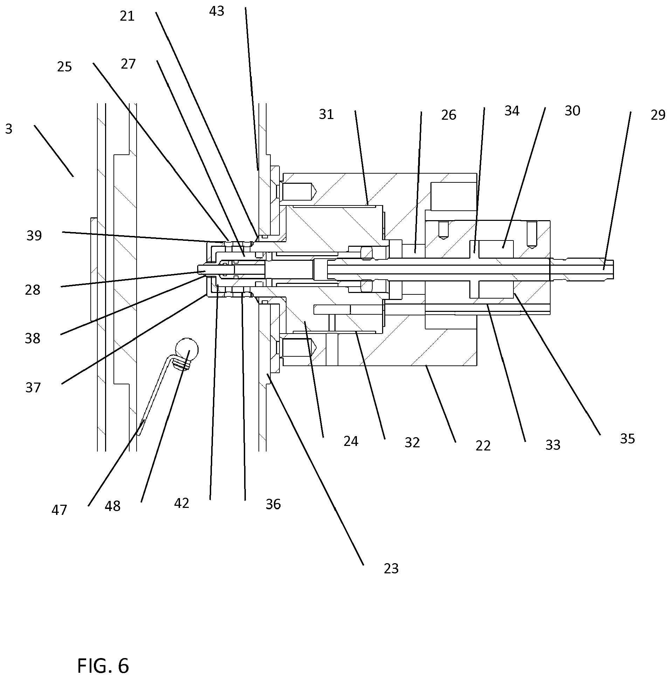

When the measurement process is concluded, the computer instructs the electrical power source to reverse polarity over the Peltier-elements 31, 32. They are activated to begin heating of the inner housing part 24 until the chocolate sample 44 is soft or even liquid. The piston 27 is then activated to travel in the channel 26 to the inner position disclosed in figure 6, where the inner stern surface 42 of the piston 27 is at the stern part 37 of the inner housing part 24 at the same time squeezing the sample chocolate 44 out through the plurality of openings 39 in the inner housing 24. The sample 44 mixes nicely up into the flow of tempered chocolate mass in the conduit part 3.

The inventive apparatus provides the available measurement chamber 36 in the inner of the conduit part 3, so that the sample 44 is disposed fast and effectively out into the flow of chocolate in the conduit part without the use of any further mechanical apparatus means or installations and without the necessity of moving the sample to another position before disposing it. The inventive solution is simple and performs fast and effective.

It is especially surprising that the measurement chamber can be maintained fully in the flow of chocolate in the conduit as the sample is being cooled and temperature measured. Then, the inventive solution provides the unique advantage, that the sample can be heated in the same position and disposed directly out into the flow of chocolate in the conduit, only by the return travel of the piston. The inventive apparatus is having an unforeseen effective measurement and melting chamber for the chocolate samples.

The inventive apparatus 1 is also especially fast and easy to fit-in and montage in a pre-made opening 21 in the chocolate conduit 3 or 17.

A flow-diverging plate 47 is arranged in the conduit 3 or 17 up-stream of the apparatus 1 and is hinged at one end to an axle 48 extending transversely through the conduit part 3 or 17 and the opposite wall parts thereof.

When turning the axle the flow diverging plate 47 is positioned in a desired angular position. Then the flow of tempered chocolate mass in the conduit 3 is increased and directed towards the inner housing part 24 if so desired The increased flow is flushing even more effectively through the plurality of openings 39 in the inner housing part 24. This is especially an advantage when the chocolate mass has a high viscosity such as milk chocolate, white chocolate, nougat or filling mass for pralines or like mass with a high content of ingredients in more course particle form.
- 1:: apparatus for determining crystallization solidification curves
- 2:: housing cover
- 3:: conduit part
- 4:: flange
- 5:: flange
- 6:: clamp
- 7:: bolts
- 8:: nuts
- 9:: pressurized air connection
- 10:: pressurized air connection
- 11:: multi-connection
- 12:: knob
- 13:: mantle for water
- 14:: tempering apparatus
- 15:: touch-screen
- 16:: flange
- 17:: chocolate conduit
- 18:: lower flange
- 19:: chocolate conduit
- 20:: flow direction
- 21:: opening
- 22:: outer housing part
- 23:: outer periphery of conduit
- 24:: inner housing part
- 25:: end
- 26:: channel
- 27:: piston
- 28:: temperature sensor
- 29:: bore
- 30:: chamber
- 31:: upper Peltier-element
- 32:: lower Peltier-element
- 33:: stop
- 34:: ring part
- 35:: end surface
- 36:: measurement chamber
- 37:: stern part
- 38:: central opening in stern part
- 39:: plurality of openings
- 40:: electrical power source
- 41:: external source of pressurized air
- 42:: inner stern surface of piston
- 43:: inner surface of conduit
- 44:: sample
- 45:: inside of inner housing
- 46:: Temper-curve
- 47:: flow-diverging plate
- 48:: axle

## Claims

1. A unit comprising an apparatus (1) for determining crystallization solidification curves of chocolate mass and other fat masses and a supply conduit part (3) with flanges (4, 5), the apparatus (1) being arranged onto an opening (21) in the supply conduit part (3) with the flanges (4, 5) adapted to be installed in a chocolate mass conduit (17, 19) for tempered chocolate mass from a tempering apparatus (14),
the apparatus (1) comprising an outer housing part (22) arranged at the outer periphery (23) of the supply conduit part (3) and an inner housing part (24) extending in through the opening (21) in radial direction towards the center of the conduit part (3), in which conduit part (3) the end (25) of the inner housing part (24) is arranged,
a channel (26) extending in radial direction from the outer housing (22) and
into the inner housing (24), a piston (27) arranged in the channel (26),
a temperature sensor (28) arranged in the piston (27),
a pressure source (30) arranged at the outer housing part (22) adapted for controlling the movements of the piston (27), when a sample of chocolate mass is to be drawn into a measurement chamber (36) in the channel (26) or when the sample is to be exposed after temperature measurement,
a tempering source (31, 32) adapted for cooling of the inner housing part (24) under temperature measurement and solidification of the sample in the chamber (36),
and a stop (33) arranged in the outer housing (22) for determining the outer position of the piston (27) in the channel (26) thereby providing the measurement chamber (36) in the channel between the piston (27) in the outer position and the end (25) of the inner housing part (24),
**characterized in that** the end (25) of the inner housing part (24) comprises a stern part (37) closing off the channel (26) apart from a central opening (38) fitted for the movement of the temperature sensor (28) in the opening (38) when moving the piston (27) forth and back in radial direction,
and that a plurality of openings (39) are arranged around the inner housing part (24) for flow of chocolate mass between the conduit part (3) and the measurement chamber (36) in the channel (26),
and that the tempering source (31, 32) is also adapted for heating of the inner housing part (24), so that a solidified sample in the measurement chamber (36) can be melted before being exposed by the piston (27) through the plurality of openings (39),and that the tempering source, which is adapted for both cooling and heating, is comprising at least one Peltier Element (31, 32).

2. Unit according to claim 1, **characterized in that** the stop (33) is arranged at a position in the outer housing (22), so that when the piston (27) is at the stop (33) in the outer position, the inner stern surface (42) of the piston (27) is at least at the inner surface (43) of the conduit (3) or in the conduit (3), thereby determining the full length of the measurement chamber (36) to be within the conduit (3).

3. Unit according to claim 2, **characterized in that** the full length of the measurement chamber (36) is between ¼ and ½ of the inner diameter of the conduit (3).

4. Unit according to any one of the preceding claims, **characterized in that** the at least one Peltier element (31, 32) is arranged in direct contact with the inner housing part (24).

5. Unit according to claim 4, **characterized in that** electrical power to the Peltier elements (31, 32) is controlled by a device such as a computer (15) so that pole reversing is performed when controlling from cooling to heating and vice versa.

6. Unit according to claim 5, **characterized in that** a flow diverging plate (47) is arranged in the conduit (3) part up-stream of the apparatus (1) and is hinged at one end to an axle (48) extending transversely through the conduit (3) part and the opposite wall parts thereof, at least one end of the axle being adapted to be turned so as to position the flow diverging plate (47) in a desired angular position.

7. Tempering apparatus (14) for tempering of chocolate mass or other fat mass comprising a unit according to any one of the preceding claims.

8. Use of the unit according to any one of claims 1 to 6 for milk chocolate, white chocolate, nougat or filling mass for pralines.

## Patentansprüche

1. Einheit, eine Vorrichtung (1) zum Bestimmen von Kristallisationserstarrungskurven von Schokoladenmasse und anderen Fettmassen und einen Zufuhrleitungsteil (3) mit Flanschen (4, 5) umfassend, wobei die Vorrichtung (1) auf einer Öffnung (21) in dem Zufuhrleitungsteil (3) angeordnet ist, wobei die Flansche (4, 5) dafür eingerichtet sind, in einer Schokoladenmasseleitung (17, 19) für temperierte Schokoladenmasse von einer Temperierungsvorrichtung (14) eingebaut zu sein,
wobei die Vorrichtung (1) Folgendes umfasst:
einen äußeren Gehäuseteil (22), der am Außenumfang (23) des Zufuhrleitungsteils (3) angeordnet ist, und einen inneren Gehäuseteil (24), der sich in radialer Richtung durch die Öffnung (21) hin zur Mitte des Leitungsteils (3) erstreckt, wobei in dem Leitungsteil (3) das Ende (25) des inneren Gehäuseteils (24) angeordnet ist,
einen Kanal (26), der sich von dem äußeren Gehäuse (22) in radialer Richtung und in das innere Gehäuse (24) erstreckt, wobei in dem Kanal (26) ein Kolben (27) angeordnet ist,
einen Temperatursensor (28), der in dem Kolben (27) angeordnet ist,
eine Druckquelle (30), die an dem äußeren Gehäuseteil (22) angeordnet und dafür eingerichtet ist, die Bewegungen des Kolbens (27) zu steuern, wenn eine Probe von Schokoladenmasse in eine Messkammer (36) in dem Kanal (26) zu ziehen ist oder wenn die Probe nach der Temperaturmessung freizugeben ist,
eine Temperierungsquelle (31, 32), die zum Kühlen des inneren Gehäuseteils (24) während der Temperaturmessung und des Erstarrens der Probe in der Kammer (36) eingerichtet ist,
und einen Anschlag (33), der in dem äußeren Gehäuse (22) angeordnet ist, um die äußere Position des Kolbens (27) in dem Kanal (26) zu bestimmen, wodurch die Messkammer (36) in dem Kanal zwischen dem Kolben (27) in der äußeren Position und dem Ende (25) des inneren Gehäuseteils (24) bereitgestellt wird,
**dadurch gekennzeichnet, dass** das Ende (25) des inneren Gehäuseteils (24) einen Schaftteil (37) umfasst, der den Kanal (26) bis auf eine mittige Öffnung (38), die für die Bewegung des Temperatursensors (28) in der Öffnung (38) eingepasst ist, abschließt, wenn sich der Kolben (27) in radialer Richtung vor und zurück bewegt,
und dass für das Fließen von Schokoladenmasse zwischen dem Leitungsteil (3) und der Messkammer (36) in dem Kanal (26) mehrere Öffnungen (39) um den inneren Gehäuseteil (24) herum angeordnet sind,
und dass die Temperierungsquelle (31, 32) außerdem für das Erwärmen des inneren Gehäuseteils (24) eingerichtet ist, so dass eine erstarrte Probe in der Messkammer (36) geschmolzen werden kann, bevor sie von dem Kolben (27) durch die mehreren Öffnungen (39) hindurch freigegeben wird, und dass die Temperierungsquelle, die für sowohl ein Kühlen als auch ein Erwärmen eingerichtet ist, mindestens ein Peltier-Element (31, 32) umfasst.

2. Einheit nach Anspruch 1, **dadurch gekennzeichnet, dass** der Anschlag (33) an einer Position in dem äußeren Gehäuse (22) angeordnet ist, so dass, wenn sich der Kolben (27) in der äußeren Position an dem Anschlag (33) befindet, die innere Schaftfläche (42) des Kolbens (27) sich mindestens an der Innenfläche (43) der Leitung (3) oder in der Leitung (3) befindet, wodurch die volle Länge der Messkammer (36) als in der Leitung (3) befindlich bestimmt wird.

3. Einheit nach Anspruch 2, **dadurch gekennzeichnet, dass** die volle Länge der Messkammer (36) zwischen ¼ und ½ des Innendurchmessers der Leitung (3) beträgt.

4. Einheit nach einem der vorhergehenden Ansprüche, **dadurch gekennzeichnet, dass** das mindestens eine Peltier-Element (31, 32) in direktem Kontakt mit dem inneren Gehäuseteil (24) angeordnet ist.

5. Einheit nach Anspruch 4, **dadurch gekennzeichnet, dass** Elektroenergie zu den Peltier-Elementen (31, 32) durch ein Gerät, wie etwa einen Computer (15), gesteuert wird, so dass eine Polumkehrung ausgeführt wird, wenn vom Kühlen auf Erwärmen und umgekehrt gesteuert wird.

6. Einheit nach Anspruch 5, **dadurch gekennzeichnet, dass** in dem Leitungsteil (3) prozessaufwärts der Vorrichtung (1) eine Stromteilungsplatte (47) angeordnet und an einem Ende an einer Achse (48) angelenkt ist, die sich quer durch den Leitungsteil (3) und die gegenüberliegenden Wandteile desselben erstreckt, wobei mindestens ein Ende der Achse dafür eingerichtet ist, derart gedreht zu werden, dass die Stromteilungsplatte (47) in einer gewünschten Winkelposition positioniert wird.

7. Temperierungsvorrichtung (14) zum Temperieren von Schokoladenmasse oder anderer Fettmasse, eine Einheit nach einem der vorhergehenden Ansprüche umfassend.

8. Verwendung der Einheit nach einem der Ansprüche 1 bis 6 für Milchschokolade, weiße Schokolade, Nougat oder Füllmasse für Pralinen.

## Revendications

1. Unité comprenant un appareil (1) pour déterminer des courbes de solidification par cristallisation d'une masse de chocolat et d'autres masses grasses et une partie de conduite d'alimentation (3) ayant des rebords (4, 5), l'appareil (1) étant agencé sur une ouverture (21) dans la partie de conduite d'alimentation (3) ayant les rebords (4, 5) conçue pour être installée dans une conduite de masse de chocolat (17, 19) pour une masse de chocolat tempérée à partir d'un appareil de tempérage (14),
l'appareil (1) comprenant une partie de boîtier externe (22) agencée à la périphérie externe (23) de la partie de conduite d'alimentation (3) et une partie de boîtier interne (24) s'étendant à travers l'ouverture (21) dans la direction radiale vers le centre de la partie de conduite (3), partie de conduite (3) dans laquelle l'extrémité (25) de la partie de boîtier interne (24) est agencée,
un canal (26) s'étendant dans la direction radiale à partir du boîtier externe (22) et dans le boîtier interne (24), un piston (27) étant agencé dans le canal (26),
un capteur de température (28) agencé dans le piston (27), une source de pression (30) agencée au niveau de la partie de boîtier externe (22) conçue pour commander les mouvements du piston (27), lorsqu'un échantillon de masse de chocolat doit être aspiré dans une chambre de mesure (36) dans le canal (26) ou lorsque l'échantillon doit être exposé après une mesure de température,
une source de tempérage (31, 32) conçue pour le refroidissement de la partie de boîtier interne (24) au cours d'une mesure de température et de la solidification de l'échantillon dans la chambre (36),
et une butée (33) agencée dans le boîtier externe (22) pour déterminer la position externe du piston (27) dans le canal (26), ce qui permet de disposer la chambre de mesure (36) dans le canal entre le piston (27) à la position externe et l'extrémité (25) de la partie de boîtier interne (24),
**caractérisée en ce que** l'extrémité (25) de la partie de boîtier interne (24) comprend une partie arrière (37) fermant le canal (26) à distance d'une ouverture centrale (38) adaptée pour le mouvement du capteur de température (28) dans l'ouverture (38) lors du déplacement du piston (27) vers l'avant et vers l'arrière dans la direction radiale,
et qu'une pluralité d'ouvertures (39) sont agencées autour de la partie de boîtier interne (24) pour l'écoulement d'une masse de chocolat entre la partie de conduite (3) et la chambre de mesure (36) dans le canal (26),
et que la source de tempérage (31, 32) est également conçue pour le chauffage de la partie de boîtier interne (24) de telle sorte qu'un échantillon solidifié dans la chambre de mesure (36) puisse être fondu avant d'être exposé par le piston (27) à travers la pluralité d'ouvertures (39) et que la source de tempérage, qui est conçue à la fois pour le refroidissement et le chauffage comprenne au moins un élément Peltier (31, 32).

2. Unité selon la revendication 1, **caractérisée en ce que** la butée (33) est agencée à une position dans le boîtier externe (22) de telle sorte que, lorsque le piston (27) se trouve au niveau de la butée (33) à la position externe, la surface arrière interne (42) du piston (27) soit au moins au niveau de la surface interne (43) de la conduite (3) ou dans la conduite (3), ce qui permet de déterminer que la longueur totale de la chambre de mesure (36) se trouve à l'intérieur de la conduite (3) .

3. Unité selon la revendication 2, **caractérisée en ce que** la longueur totale de la chambre de mesure (36) fait entre 1/4 et 1/2 du diamètre interne de la conduite (3).

4. Unité selon l'une quelconque des revendications précédentes, **caractérisée en ce que** le ou les éléments Peltier (31, 32) sont agencés en contact direct avec la partie de boîtier interne (24).

5. Unité selon la revendication 4, **caractérisée en ce que** de l'énergie électrique fournie aux éléments Peltier (31, 32) est régulée par un dispositif tel qu'un ordinateur (15) de telle sorte qu'une inversion de pôles est réalisée lors de la commande passant du refroidissement au chauffage et vice versa.

6. Unité selon la revendication 5, **caractérisée en ce qu'**une plaque de divergence de l'écoulement (47) est agencée dans la partie de conduite (3) en amont de l'appareil (1) et est articulée au niveau d'une extrémité à un axe (48) s'étendant transversalement à travers la partie de conduite (3) et les parties de paroi opposées de ce dernier, au moins une extrémité de l'axe étant conçue pour être tournée de sorte à positionner la plaque de divergence de l'écoulement (47) à une position angulaire souhaitée.

7. Appareil de tempérage (14) pour le tempérage d'une masse de chocolat ou d'une autre masse grasse comprenant une unité selon l'une quelconque des revendications précédentes.

8. Utilisation de l'unité selon l'une quelconque des revendications 1 à 10 pour du chocolat au lait, du chocolat blanc, du nougat ou une masse de remplissage pour des pralines.
